(19) Europäisches Patentamt
European Patent Office
Office européen des brevets

(11) Publication number: 0 097 952 B1

(12) EUROPEAN PATENT SPECIFICATION

(45) Date of publication of patent specification: 12.11.86

(21) Application number: 83106271.6

(22) Date of filing: 28.06.83

(51) Int. Cl.$^4$: G 01 N 33/52, G 01 N 33/533, G 01 N 33/549

# (54) Multilayer analytical element.

(30) Priority: 30.06.82 JP 114359/82

(43) Date of publication of application: 11.01.84 Bulletin 84/02

(45) Publication of the grant of the patent: 12.11.86 Bulletin 86/46

(84) Designated Contracting States: DE GB

(56) References cited:
EP-A-0 051 183
EP-A-0 066 648
US-A-4 270 920
US-E- 30 267

(73) Proprietor: FUJI PHOTO FILM CO., LTD.
210 Nakanuma Minamiashigara-shi
Kanagawa-ken, 250-01 (JP)

(72) Inventor: Nagatomo, Shigeru
c/o Fuji Photo Film Co., Ltd. 3-11-46, Senzui
Asaka-shi Saitama (JP)
Inventor: Yasuda, Yukio
c/o Fuji Photo Film Co., Ltd. 3-11-46, Senzui
Asaka-shi Saitama (JP)
Inventor: Masuda, Nobuhito
c/o Fuji Photo Film Co., Ltd. 3-11-46, Senzui
Asaka-shi Saitama (JP)
Inventor: Makiuchi, Hajime
c/o Fuji Photo Film Co., Ltd. 3-11-46, Senzui
Asaka-shi Saitama (JP)

(74) Representative: Werner, Hans-Karsten, Dr. et al
Deichmannhaus am Hauptbahnhof
D-5000 Köln 1 (DE)

EP 0 097 952 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

This invention relates to a multilayer analytical element for fluorometric assay or fluorometric quantitative analysis of an analyte contained in a sample liquid. More particularly, this invention relates to a multilayer analytical element appropriately employable for fluorometric measurement of concentration of substances which play important roles in biochemical process or of various substances derived from the living body or organisms (referred to hereinafter as "specific component") using proteins specifically bindable to the substances through competitive binding reactions. This invention further relates to a fluorometric assay employing said multilayer analytical element.

Development of the Invention

In clinical examination, a variety of methods are known where several reagent solutions are added to a testing liquid and the concentration of a specific component in the sample liquid is measured. In these methods, the reaction reagents are necessarily weighed with high accuracy and thoroughly mixed in order to improve the reproducibility of the measurements and, in some occasions, separation of the resulting precipitates from the supernatants through centrifugation is required. Thus, these conventional methods inherently require highly trained skills.

Accordingly, certain improved apparatus in which the required procedures are all automated have been proposed so as to replace the above-mentioned conventional method. These automated apparatuses are able to provide high reproducibility without such high skills and are convenient for measuring large numbers of sample liquids. Nevertheless, these have serious disadvantages such as high expense. In order to obviate the disadvantages of such automated apparatus, a multilayer analytical elements in which reaction reagents are in advance incorporated have been proposed as a simple analytical means where supply of the reagent solutions is substantially not required, whereby no trained skills are required. These multilayer analytical elements are advantageous in that they are inexpensive as compared with the automated apparatus. However, although various improvements have been proposed with respect to the multilayer analytical elements utilizing chemical reactions or enzymatic reactions, it is still difficult to assay trace components or components having high structural specificity.

For the analysis of such components using reagent solutions, there is known a method utilizing an immunological reaction, that is, an immunoassay. However, only a few proposals have been made until now with respect to a multilayer analytical element in the form of a film in which the reagents for an immunological reaction are incorporated. Most of the conventional multilayer analytical elements find difficulty in obtaining satisfactory analytical results where they are employed for assay involving an immunological reaction. This is because the immunological reaction inherently has specific problems.

For obtaining satisfactory analytical results in the immunoassay, it is very important to introduce a minimum amount of a sample liquid into the multilayer analytical element within a relatively short period, as well as to hold the so introduced sample liquid within the element for a sufficient period to carry out the desired antigen-antibody reaction.

From the above-mentioned viewpoints, the present inventors have studied the previously proposed multilayer analytical elements such as those described in Japanese Patent Publication No. 53(1978)—21677 corresponding to U.S. Patent 3,992,158; Japanese Patent Provisional Publication No. 51(1976)—40191 corresponding to U.S. Patent 4,042,335; Japanese Patent Provisonal Publication No. 56(1981)—24576; Japanese Patent Provisional Publication No. 55(1980)—90859 corresponding to U.S. Patent 4,258,001; and Japanese Patent Provisional Publication No. 53(1978)—131089 corresponding to U.S. Patent 4,144,306.

From these studies, the present inventors have noted that the previously known multilayer analytical elements subjected to their study all are unsatisfactory in their capacity for holding the sample liquid within the element. Thus, these multilayer analytical elements show serious problems in the accuracy, sensitivity and rapidity in carrying out the immunoassay.

The present inventors already invented a multilayer analytical element comprising an integrated detection member which comprises a reaction layer for carrying out a reaction between a specific component (analyte) and a protein specifically bindable to the analyte, and a detection layer receiving a product showing a signal modified in response to the concentration of the analyte. This invention was applied for European Patent under Application Number 81108365.8 on October 15, 1981 (published under EP 0 066 648 A1). The multilayer analytical element according to the above-mentioned invention has successively solved the problem with respect to holding the sample liquid within the element. In this multilayer analytical element, a competitive antigen-antibody reaction is carried out in the reaction layer. Until the reaction is complete, the resulting bound labelled complex (B) remains in the reaction layer, while the unreated free labelled substance (F) diffuses into the detection layer placed under the reaction layer, in which the free substance (F) is photometrically detected.

The above-mentioned analytical element enables a sufficient amount of a sample liquiud to participate in the competitive antigen-antibody reaction as compared with the conventional multilayer analytical films, and thus assures high analytical reproducibility at high sensitivity. Accordingly, this element has been thought to solve the aforementioned problems, that is, difficulty in providing a rapid and simple analytical means, inherently attached to the immunoassay in the dry system, by enabling separation between the

bound labelled complex (B) and the free labelled substance (F), namely B/F separation, simultaneously with the progress of the antigen-antibody reaction in the multilayer analytical element.

The necessity of the B/F separation has caused various difficulties in the immunoassay. In the first place, B/F separation to a satisfactory level is as such rather difficult, and requires a lengthy period. In the second place, the B/F separation procedure is difficultly incorporated into an automated continuous analytical process. Also difficult is the accomplishment of B/F separation under an equilibrium state. Such difficulties in the B/F separation not only require trained high skills in the analytical procedure, but also reduce the reliability of the analytical data. For coping with these problems, Japanese Patent Provisional Publications Nos. 53(1978)—70924 and 53(1978)—38619 disclose analytical processes in which the fluorescence of the labelled substance is determined in the reaction mixture without B/N separation, that is, no separation between the bound labelled complex (B) and the free labelled substance (F) is involved. However, these analytical processes are only applicable to a specific system where the fluorescence of the bound labelled complex (B) is substantially stronger or weaker than that of the free labelled substance (F), and are not widely applicable to the normal or ordinary antigen-antibody reaction. Other disadvantageous features of these analytical processes comprising no B/F separation resides in the detection of background fluorescence, thereby lowering the S/N ratio. Also disadvantageous is a need of trained high skills in, for instance, measuring the reagent solutions involved. Although such measuring procedure can be incorporated into the automated apparatus to simplify the total procedure, such apparatus becomes highly expensive.

The present inventors have discovered that the defective features of the conventional arts can be effectively overcome by a multilayer analytical element comprising specifically arranged members so as to carrying out the B/F separation within the analytical element.

Summary of the Invention

A primary object of the present invention is to provide a multilayer analytical element with reduced noise, namely, with high S/N ratio.

Another object of the invention is to provide a multilayer analytical element for fluorometric assay in which the B/F separation is done within the element and the signal of the bound labelled complex (B) can be directly determined.

The present invention provides a

multilayer analytical element for fluorometric assay of an analyte contained in an aqueous sample liquid, comprising:

a porous fibrous or non-fibrous spreading sheet containing a predetermined amount of a fluorescent-labelled analyte substance or analogue thereof;

a porous sharing sheet; and

a reaction sheet containing a predetermined amount of a protein fixed therein which is specifically bindable to the analyte; and the analogue thereof;

in which a ratio of liquid-holding capacity between the porous sharing sheet and the reaction sheet is within the range of 10:1 to 0.5:1, and these three sheets are laminated in this order.

Furthermore, the present invention is directed to

a slide for fluorometric assay of an analyte contained in an aqueous sample liquid, in which said multilayer analytical element is provided in a frame having an opening for introduction of the sample liquid and an opening for enabling fluorometric measurement.

Moreover, the invention is directed to a fluorometric assay of an analyte contained in an aqueous sample liquid which is characterized by employing said multilayer analytical element and accomplishing a substantial separation of the bound fluorescent-labelled complex (B) from the free fluorescent-labelled analyte substance or analogue thereof (F) through sharing of the free fluorescent-labelled analyte substance or analogue thereof (F) between the reaction sheet and a space other than the reaction sheet.

Brief Description of the Drawings

Figure 1 is a sectional view showing the essential layer structure of a multilayer analytical element of the invention. Figure 1a is a perspective view of the multilayer analytical element shown in Figure 1.

Figure 2 is a vertical sectional view of an embodiment of a slide for fluoroimmunoassay according to the present invention. Figure 2a is a top plane view of the slide shown in Figure 2. Figure 2b is a sectional view of a upper part of the slide taken along line I—I in Figure 2a. Figure 2c is a bottom plane view of the slide shown in Figure 2. Figure 2d is a sectional view of a lower part of the slide taken along line I—I in Figure 2c.

Figure 3 is a vertical sectional view of another embodiment of a slide for fluoroimmunoassay according to the present invention. Figures 3a and 3b are respective sectional views of two frames. Figures 3c and 3b are a sectional view and plane view, respectively, of the distributor.

Figure 4 is a vertical sectional view of a further embodiment of a slide for fluoroimmunoassay according to the present invention. Figures 4a and 4b are respectively a sectional view and a plane view of the transparent support.

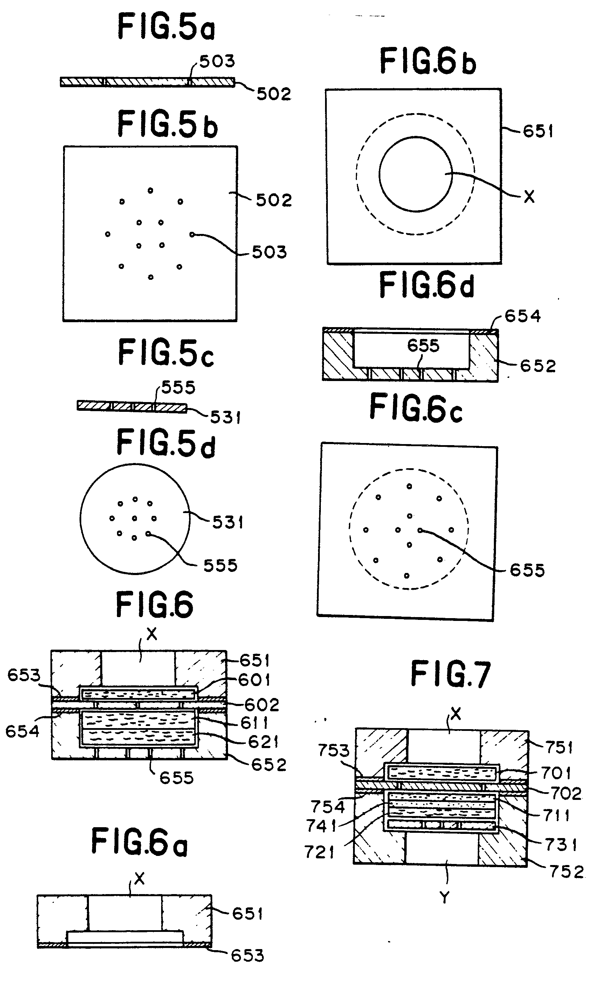

Figure 5 is a vertical sectional view of a still further embodiment of a slide for fluoroimmunoassay according to the present invention. Figures 5a and 5b are respectively a sectional view and a plane view of a

distributor having openings for supplying a sample liquid. Figures 5c and 5d are respectively a sectional view and a plane view of the transparent support having vents.

Figure 6 is a vertical sectional view of a still further embodiment of a slide for fluoroimmunoassay according to the present invention. Figures 6a and 6b are respectively a sectional view and a top plane view of the frame having an opening for introduction of a sample liquid. Figures 6c and 6d are respectively a sectional view and a bottom plane view of the lower transparent frame having vents.

Figure 7 is a vertical sectional view of a still further embodiment of a slide for fluoroimmunoassay according to the present invention.

Preferred Embodiments of the Invention

In the present invention, the term "analyte substance" means the same substance as an analyte. For instance, if the analyte in the sample liquid is thyroxine, a predetermined amount of a fluorescent-labelled analyte substance, that is, a fluorescent-labelled thyroxine, is subjected to the antigen-antibody reaction in the presence of the unknown amount of the analyte (thyroxine). An analogue of the analyte substance can also be subjected to the antigen-antibody reaction in the same manner to carry out the fluorometric immunoassay. Accordingly, the term "analyte substance" used hereinunder is meant to include an analyte substance and an analogue thereof, unless otherwise specified.

For simplicity the term "bound fluorescent-labelled complex (B)" used in the following shall be understood as a complex formed on a fluorescence-labelled compound with its receptor under formation of a fluorescing complex.

The analytical element of the present invention is employable for fluorometric assay of an analyte contained in an aqueous sample liquid, in which the assay comprises

a stage of subjecting the analyte, a predetermined amount of a fluorescent-labelled analyte substance, and a predetermined amount of a protein specifically bindable to the analyte to competitive binding reaction;

a stage of substantially separating the resulting bound fluorescent-labelled complex (B) of the protein (including the resulting bound fluorescent-labelled complex (B) of the protein and the analyte, as well as the resulting bound fluorescent-labelled complex (B) of the protein and the analyte substance) from the free fluorescent-labelled analyte substance (F); and

a stage of determining the fluorescence strength of said labelled complex (B).

In the multilayer analytical element, the abovementioned substantial separation of the bound fluorescent-labelled complex (B) of the protein from the free fluorescent-labelled analyte substance (F), namely B/F separation, is achieved by the porous sharing sheet (b), occasionally referred to simply as sharing sheet, provided therein.

The sharing sheet functions for sharing the free fluorescent-labelled substance (F) into other space than the space of a reaction sheet containing a predetermined amount of the protein specifically bindable to the analyte fixed therein.

In the present invention, the term "sharing" means "introducing the free fluorescent-labelled substance (F) into a space other than the space of the reaction sheet". As will be described hereinafter, the multiple layers of the analytical element of the invention can be so designed that the free fluorescent-labelled substance (F) can be distributed in the sharing sheet as much as possible, whereby most of the free fluorescent-labelled substance (F) is introduced into a space other than the space of the reaction sheet, while the reaction sheet contains substantially only the bound fluorescent-labelled complex (B). Therefore, the B/F separation is sufficiently achieved, and the fluorescence strength of the bound fluorescent-labelled complex (B) can be independently measured.

Accordingly, the term "separation" used in the present invention means the separation of bound fluorescent-labelled complex (B) from a free fluorescent-labelled substance (F) resulting from:

the first stage in which an applied sample liquid is shared over the multilayer analytical element at a certain amount per unit area and the analyte in the sample liquid is uniformly shared over the analytical element, as well as the fluorescent-labelled analyte substance contained in the spreading sheet is uniformly shared over the analytical element through dissolution in the medium of the sample liquid; and

the second stage in which the analyte and the fluorescent-labelled analyte substance are competitively reacted in the reaction sheet to a specifically bindable protein previously fixed to the reaction sheet, resulting in production of bound fluorescent-labelled complex (B) within the reaction sheet.

The above-mentioned first and second stages are not thought to occur independently from each other, that is, these phenomena mentioned in both stages are thought to take place under microscopically mixed conditions immediately after dropping or spotting a sample liquid on the analytical element, and after lapse of a certain period (incubation period) the B/F separation reaches a totally sufficient level.

The reaction sheet provides a space for carrying out the competitive reaction between the fluorescent-labelled analyte substance and the analyte for the specifically bindable protein fixed therein. As a result of the competitive reaction, photometrically detectable bound fluorescent-labelled complex (B) is produced in the reaction sheet. The complex (B) is thus produced under substantial separation from the free (unreacted) fluorescent-labelled substance (F) shared in the sharing sheet, namely, a space different from the reaction sheet. The term "substantial separation" includes a case in which the bound fluorescent-labelled complex (B) is present only in the reaction sheet and most of the free fluorescent-labelled substance (F) is present in

a space other than the reaction sheet, such as the spreading sheet and the sharing sheet upon sharing, while a portion of the substance (F) still remains in the reaction sheet. Accordingly, the fluorescence strength value measured on the reaction sheet includes not only the fluorescence strength of the bound complex (B), but also that of the free fluorescent-labelled substance (F) present in the reaction sheet. Thus measured fluorescence strength value is plotted against a predetermined calibration curve to determine the amount of the analyte.

If the analytical element is so designed as to increase the sharing ratio for space other than the reaction sheet, the influence on the fluorescence strength given by the free fluorescent-labelled substance (F) can be reduced to a neglectable level. Accordingly, the so designed element is a preferred embodiment of the present invention.

The sharing ratio of the free fluorescent-labelled substance (F) between the reaction sheet and other space than the reaction sheet depends upon nature of the material, thickness and thickness ratio of the reaction sheet and one or more sheets providing the space other than the reaction sheet, affinity of the analyte and the fluorescent-labelled analyte substance to the material constituting each sheet. For instance, the sharing ratio can be adjusted by such a simple manner that each sheet is prepared to have a controlled liquid holding capacity which means a volume of a liquid which can be held within the sheet.

In the above, the liquid holding capacity is determined by multiplying the volume of the sheet by a void ratio. In the present invention, the abovementioned substantial separation is accomplished by adjusting the liquid capacity ratio between the sharing sheet and the reaction sheet to a value in the range of from 10:1 to 0.5:1. If the liquid holding capacity of the sharing sheet increase relative to that of the reaction sheet, the amount shared into the sharing sheet increases. Thus, the above-mentioned liquid holding capacity ratio preferably is in the range of from 5:1 to 1:1. If the liquid holding capacity is less than 1, the B/F separation is not effectively accomplished and the S/N ratio is kept at a low level. On the other hand, various difficulties reside in preparation of a multilayer analytical element having a liquid holding capacity more than 10.

If the material of the sharing sheet is the same as that of the reaction sheet, the liquid holding capacity ratio is generally determined according to the thickness of each sheet. However, even if the ratio in thickness between the sharing sheet and the reaction sheet is equivalent to 1:1, the sharing degree can be increased to provide a greater amount of the free fluorescent-labelled substance (F) into the sharing sheet by, for instance, increasing the liquid holding capacity per thickness of the sharing sheet as compared with that of the reaction sheet, or incorporating a protein having a lower binding constant which is different from the protein fixed in the reaction sheet, into the sharing sheet.

The sharing sheet is provided between the spreading sheet and the reaction sheet and made of a porous fibrous material.

Examples of the porous fibrous material include natural fibers such as pulp, cotton, silk, and wool: semisynthetic fibers such as cellulose esters and viscose rayon; synthetic fibers such as polyamides (e.g., nylon 6, nylon 66, and nylon 610), polyesters (e.g., polyethylene terephthalate), and polyolefins (e.g., polypropylene); and fibrous inorganic materials such as glass fibers, colored glass fibers, and asbestos. These materials can be employed in such forms as fabrics (woven or knitted), felts, and non-woven fabrics. Also employable are water permeable papers.

As mentioned above, the fabrics and others employable for the sharing sheet can be made of a variety of materials including natural fibers such as plant origin fibers, animal origin fibers and mineral origin fibers and synthetic fibers such as inorganic, regenerated, semisynthetic and synthetic fibers. Among the their forms, a plain weave which is produced by weaving alternatively warp and weft yarns is preferably employed. The warp and weft both are preferably in the count ranges of from 20 to 120. Particularly preferred are cotton fabrics such as so-named close cloth, canequim, broadcloth and poplin. Moreover, mixed fabrics composed of cotton and other natural fibers (e.g., kapok, flax, hemp, ramie, and silk) or synthetic fibers (e.g., viscose rayon, copper-ammonia rayon, cellulose acetate, cellulose triacetate, polyvinyl alcohol and polyethylene terephthalate) in the above-mentioned woven forms. Also employable are fabrics of chemical fibers in these woven forms.

The paper employable as the sharing sheet may be any of various water permeable papers. For instance, a filter paper can be employed, and a thick filter paper being loose in weave is preferred. The Indian paper and Japanese paper such as paper mulberry or Mitsumata can be also employed. Moreover, the sharing sheet can be a water permeable synthetic paper prepared from synthetic polymer fibers, an asbestos sheet, a glass fiber sheet.

The above-described material can be employed as the sharing sheet after incorporating fine particles therein. These fine particles serve as fillers for filling in part the voids of the porous fibrous material to render the manner of permeation of a liqud anisotropic. Examples of the fine particles employable for these purposes include fine particles of non-fibrous polymers of polysaccharides such as agar, agarose, Sepharose®, Sephadex® and dextran, polyacrylamide, cellulose (powder), and latex prepared by polymerization or copolymerization of polymerizable ethylenic polymers. Such non-fibrous fine particles as agar, agarose, and sepharose are able to increase the liquid holding capacity per thickness of the sheet, thereby enabling enhancement of the sharing ability without increase of the thickness of the sharing sheet. The fine particles can be incorporated into the sharing sheet in an amount of up to approximately 90% by weight based on the total amount of the so incorporated sheet.

Another measure for enabling enhancement of the sharing ability without increase of the thickness of the sharing sheet is as follows: A protein such as serum albumin or serum globulin having a binding power to the fluorescent-labelled substance such as in a binding constant ranging from 10 to $10^7$ 1/mol which is less than the binding power of the protein fixed in the reaction sheet is physically or chemically fixed in the sharing sheet, thereby increasing sharing of the free fluorescent-labelled substance (F) into the sharing sheet. The procedure for fixing the protein in the sharing sheet can be carried out in the same manner as in the procedure for fixing the protein in the reaction sheet.

The thickness of the sharing sheet is determined according to the desired distribution degree, and simply determined in view of the liquid holding capacity. Generally, the thickness of the sharing sheet ranges from approx. 100 μm to approx. 2,000 μm, preferably from 200 μm to 1,000 μm.

The spreading sheet is made of a porous fibrous or non-fibrous material and provided on the sharing sheet.

The spreading sheet can contain a certain amount of a fluorescent-labelled analyte substance. In this embodiment, the spreading sheet serves for spreading substantially uniformly a sample liquid containing an analyte and simultaneously for spreading uniformly the fluorescent-labelled analyte substance dissolved in the sample liquid.

For introducing the sample liquid containing the dissolved fluorescent-labelled substance into the adjoining sharing sheet and reaction sheet, the following is required: The sample liquid applied or spotted on the spreading sheet is spread rapidly over the surface of the spreading sheet, and at the same time, a variety of reagents contained in the spreading sheet such as a fluorescent-labelled analyte substance and a buffer agent are dissolved in the sample liquid. Subsequently, the sample liquid gradually permeates into the adjoining sharing sheet and reaction sheet through capillary action provided by these porous material sheets. If the liquid absorbing power through the capillary action provided by the sharing sheet and reaction sheet is too strong, the reagent dissolved in the sample liquid as above is not uniformly shared within the sharing sheet and the reaction sheet. If the liquid absorbing powder is too weak, the desired even sharing of the reagent hardly is accomplished. Accordingly, the above-mentioned function of the spreading sheet can be obtained if a porous material sheet having an absorbing power via the capillary action higher than those of the sharing sheet and reaction sheet is employed as the spreading sheet.

The uniform sharing (or spreading) of the fluorescent-labelled substance within the sharing sheet and reaction sheet is easily attained if a predetermined amount of the fluorescent-labelled substance is dissolved in a predetermined amount of the sample liquid in advance of the applying or spotting of the sample liquid.

Since the spreading sheet is provided for uniformly spreading the liquid, the applied or spotted liquid is preferably supplied into the reaction sheet without being retained in the spreading sheet. Particularly, if the sample liquid is in a very small amount, the amount of the liquid retained in the spreading sheet is preferably reduced to a level as low as possible. For this reason, at least 70% of the applied or spotted sample liquid is preferably supplied into the sharing sheet and reaction sheet. In other words, the amount of the sample liquid retained in the spreading sheet preferably is not more than 30% of the whole liquid.

Representative examples of the material employable for the spreading sheet satisfying the above-mentioned conditions include an isotropically porous non-fibrous material and a porous fibrous material.

Examples of the isotropically porous non-fibrous material include blushed polymers (generally called "membrane filter"); materials containing micro porous particles such as diatomaceous earth and micro-crystalline particles (e.g., micro-crystalline cellulose); porous materials containing micro spherical beads of glass and synthetic polymers under point-to-point contact with binder; and blushed polymers containing fine powder of $TiO_2$ or $BaSO_4$ substantially uniformly dispersed therein.

As the examples of the porous fibrous material, there are mentioned the materials described hereinbefore for the sharing sheet.

Preferred materials for the spreading sheet are fabrics belonging to the porous fibrous material. Particularly, water-washed fabrics and hydrophilically processed fabrics are preferred for the spreading sheet material. The hydrophilically processed fabrics may be fabrics treated with water-washing, defatting and drying procedures; or fabrics treated with water-washing, defatting and drying procedures and then impregnated with a small amount of a surface active agent, a wetting agent, a hydrophilic polymer of a fine powder such as $TiO_2$ or $BaSO_4$. The arts for employing the hydrophilically processed fabrics as the spreading sheet and details of the fabrics are described in Japanese Patent Provisional Publication No. 55(1980)—164356 (corresponding to U.S. Patent 4,292,272) and these arts can be applied to the present invention.

The spreading sheet generally has a thickness in the range of from approx. 50 μm to approx. 500 μm, preferably from approx. 80 μm to approx. 300 μm.

A predetermined amount of the fluorescent-labelled analyte substance can be incorporated into the spreading sheet in the following manner: The spreading sheet is immersed in an aqueous solution containing a predetermined amount of the analyte substance (the amount is determined depending mainly upon nature of the analyte under assay) — that is, so-called bathing treatment —, and dried under atmospheric conditions or freeze-dried. Otherwise, a fluorescent-labelled substance solution of a predetermined concentration can be dropped on a spreading sheet, allowed to impregnate within the sheet, and dried in a conventional manner such as atmospheric drying or freeze-drying.

The fluorescent-labelled analyte substance incorporated into the spreading sheet in the above-described manners can be easily released upon contact with the sample liquid to be introduced in the sample liquid, and then supplied under the uniform-spreading action of the spreading sheet into the sharing sheet and reaction sheet together with the analyte. Subsequently, the analyte and the fluorescent-labelled analyte substance supplied into the reaction sheet are reacted competitively with the specifically bindable protein fixed in the reaction sheet.

The fluorescent-labelled analyte substance can be prepared in a conventional manner by combining the analyte substance with a fluorescent compound directly or indirectly through a bifunctional compound such as an amino acid, a dicarboxylic acid, or a diamino compound. Such combining processes have been heretofore reported with respect to a variey of analytes, and are well known to those skilled in the art. For instance, the details are given in CLINICAL TEST TECHNIQUE, Vol. 4, Immunoserological Test, edited by Tadashi Kawai (Igaku Shoin, Japan), 1977, pp. 97—102; Japanese Patent Provisional Publications Nos. 53(1978)—79024 and 53(1978)—142522; Biochem, Biophys. Res. Commun. *74,* 538(1977); and Clin. Chim. Acta, *83*, 161 (1978).

The fluorescent-labelled analyte substance can be prepared in any of the conventional processes, provided that the analyte substance still retains the reactivity to the protein fixed in the reaction sheet. The indirect combination between the analyte substance and the fluorescent substance through the above-mentioned bifunctional compound is frequently utilized to assure the fixed protein of satisfactory reactivity. In the present invention, such indirectly combined fluorescent-labelled substances are named "fluorescent-labelled analyte analogue". Further known in the art is a technique in which a variety of appropriate functional groups can be introduced into the fluorescent-labelled substance at sites not participating in the binding reaction so as to effectively carry out the competition with the analyte in the binding reaction to the protein fixed in the reaction sheet, or to improve the solubility. In the present invention, the substance modified as above is also called "fluorescent-labelled analyte analogue".

Representative examples of the fluorescent compound include fluorescein isothiocyanate (FITC), methylrhodamine, umbelliferone, N-methyl-2-anilino-6-naphthalenesulfonate, and ε-[5-(dimethylamino)-naphthalene-1-sulfonyl]lysine.

The fluorescent-labelled analyte substance is bound to the specifically bindable protein fixed in the reaction sheet in the course of the competitive binding reaction to produce a bound fluorescent-labelled complex (B). In the conventional fluoroimmunoassay, fluorescence strength of the bound fluorescent-labelled complex (B) or the free fluorescent-labelled substance (F) is measured.

However, it is known that in a special case the quantitative assay of an analyte can be carried out through measurement of change of the fluorescence strength between before and after the antigen-antibody reaction. For instance, it is known that the fluorescence strength of a bound fluorescent-labelled complex (B) increases after an antigen-antibody reaction in a certain system (e.g., system described in Japanese Patent Provisional Publication No. 53(1978)—79024). Also known is a reverse system in which the fluorescence strength decrease after an antigen-antibody reaction (e.g., system described in Japanese Patent Provisional Publication No. 53(1978)—38619 corresponding to U.S. Patent 4,150,949 and DE—A—27 16 276).

Either of the above-mentioned reaction systems can be applied to the present invention. Naturally, a system in which no substantial change of the fluorescence strength between and after an antigen-antibody reaction is observed can be applied to the present invention. The last mentioned system is considered to be not applicable in a fluoroimmunoassay without B/F separation.

The spreading sheet may contain the following known reagents required or preferred in fluoroimmunoassay or other reagents, in addition to the fluorescent-labelled analyte substance.

(1) Blocker agent

In certain cases, an analyte to be assayed is under engagement with polymer substances such as albumin and globulin in the serum so that the desired reaction (e.g. specific binding reaction) hardly takes place. For instance, thyroxine ($T_4$) is generally bound to albumin. In other cases, a fluorescent-labelled analyte substance is engaged with serum components so that the specifically bindable ability in the antigen-antibody reaction is reduced. For releasing or reducing such undesirable engagement between the analyte or fluorescent-labelled substance and the serum component, a reagent generally called "blocker agent" may be incorporated.

Representative examples of the blocker agent include 8-anilino-1-naphtharenesulfonic acid (ANS), Thimerosal, and salicylic acid.

(2) Buffer agent

Various buffer agents may be incorporated to provide a pH range suitable for allowing the maximum function of the blocker agent (pH 6—11); a pH range suitable for the binding reaction and the fluorometric measurement (pH 7—10); a pH range suitable for efficiently sharing the fluorescent-labelled analyte substance (pH 7—10); and others.

Examples of the buffer agent employable in the invention include known buffer agents described in CHEMICAL HANDBOOK, FUNDAMENTAL EDITION edited by the Chemical Society of Japan (Maruzen, Tokyo, 1966), pp. 1312—1320; Biochemistry, *5*(2), 467—477(1966) "Hydrogen Ion Buffers for Biological Research" by N. E. Good et al.; Analytical Chemistry, *104*, 300—310(1980) "Hydrogen Ion Buffers for

Biological Research" by W. J. Ferguson et al. More concretely, the buffer agent may be glycine, a borate, a phosphate or diethylbarbiturate.

(3) Preserving agent

The preserving agent is incorporated for keeping the specifically bindable protein from rotting, preventing moisture absorption and controlling the hygroscopic degree. As an example, sodium azide can be mentioned.

(4) Dissolving or Spreading assistant agent

Water soluble solids such as sucrose, amino acids and glycerol, or various surface active agents such as anionic surface active agents (e.g., sodium alkylbenzenesulfonate) and nonionic surface active agents (e.g., polyethylene glycol) may be incorporated for assisting dissolution of the above-mentioned reagents and further for assisting spreading of these reagents quickly and uniformly.

(5) Other substances

Bovine serum albumin (BSA), rabbit serum albumin (RSA) and gelatin are preferably incorporated for preventing pseudo reactions to assist smooth and stable progress of antigen-antibody reactions, whereby improving the accuracy and repeatability of the measurements.

Among the above-mentioned reagents and other substances, the blocker agent is preferably incorporated into the spreading sheet, while the buffer agent, preserving agent and spreading agent may be incorporated into the sharing and/or the reaction sheet according to the analytical object.

Under the sharing sheet is provided a reaction sheet.

The reaction sheet contains a predetermined amount of a specifically bindable protein fixed therein to cause a binding reaction occuring competitively between the analyte and the fluorescent-labelled analyte substance. The material of the reaction sheet is generally selected from the material given hereinbefore for the sharing sheet.

The specifically bindable protein is a protein capable of participating in the competitive binding reaction between the analyte and the fluorescent-labelled analyte substance. A representative example of the specifically bindable protein is an antibody. The specifically bindable protein is automatically determined to correspond to the analyte in view of the specific reaction concerned, and is physically (e.g., through adsorption) or chemically (e.g. through covalent or noncovalent bonding) fixed in a material constituting the reaction sheet in a conventional manner.

As the methods for incorporating the specifically bindable protein into the reaction sheet under fixing, the following methods are known:

(1) A method in which the protein is bound to a porous fibrous material by linking directly the immunoglobulin to the fibrous material through the amino groups or carboxyl groups by a physical process (e.g., by adsorption) or a chemical process (e.g., by covalent bonding) or by linking indirectly through linking components by these processes; and

(2) A method employable in the case of incorporating fine particles in which the protein is previously provided to the fine particles (e.g., agarose; otherwise, the fine particles hereinbefore given as examples of fine particles to be incorporated into the sharing sheet can be employed for this purpose) through covalent bonding or adsorption or through linking components capable of specifically binding Fc fragments (e.g., secondary antibody, protein A). Details of the fixing methods are described in Japanese Patent Provisional Publication No. 47(1972)—18597.

The protein fixed in the reaction sheet generally has a binding constant in the range of $10^7$ to $10^9$ l/mol, and is automatically determined to correspond to the analyte in view of the binding reaction to the analyte.

The protein fixed in the reaction sheet shows its specifically bindable property to an analyte or a fluorescent-labelled analyte substance upon contact with a sample liquid. The thickness of the reaction sheet generally ranges from approx. 100 µm to approx. 1,000 µm, preferably from 200 µm to 500 µm.

The multilayer analytical element of the present invention may comprise a distributor between the spreading sheet and the sharing sheet for enhancing or assisting the spreading function, in addition to the aforementioned essential three layers structure.

The distributor is a water-impermeable film having openings for supplying almost evenly the sample liquid into the lower sharing sheet and reaction sheet. The size of the opening for supply of the liquid is generally not more than 0.05 $cm^2$, preferably in the range of from 0.0001 $cm^2$ to 0.005 $cm^2$. Examples of the water-impermeable film include films of plastic materials such as cellulose esters (e.g., cellulose diacetate, cellulose triacetate, and cellulose acetate propionate), polycarbonate of bisphenol A, polyethylene terephthalate, polystyrene, polymethyl methacrylate, polyvinyl chloride, poly(vinylidene chloride — vinyl chloride), polytetrafluoroethylene, polyethylene, polypropylene, and polyamide (e.g., nylon 6); metal foils; composite films of the plastic film and a metal foil or paper; and glass plate. The thickness of the distributor generally is in the range of from approx. 20 µm to approx. 1 mm, preferably from 80 µm to 300 µm.

The multilayer analytical element of the present invention may further comprise a light shielding substance to improve the accuracy of the fluorometric measurement. For this purpose, a porous fibrous material mentioned previously as materials of the sharing sheet can be incorporated after colored with a dye having absorption band in a wavelength region of the exciting light and/or radiating fluorescence (e.g., a reactive dye, or a vat dye). Otherwise, pigment particles of white or black or colored pigments can be incorporated into the sharing sheet. Moreover, the above-mentioned dye can be fixed to the non-fibrous particles mentioned previously as materials of the sharing sheet. Also applicable is the non-fibrous

particles containing the colored pigment. By the incorporation of the light shielding substance in the manner as above, the multilayer analytical element of the present invention is given a light shielding function and advantageously employed in the photometric measurement.

Representative examples of the light shielding substance include white fine powders such as $TiO_2$ fine powder, ZnO fine powder and $BaSO_4$ fine powder, and white or pale glossy metal fine powder such as aluminum fine powder.

If the light shielding substance is incorporated into the analytical element as above, the fluorescence emitted by the serum components, of which wavelength is shorter than wavelength of the fluorescence to be measured, is absorbed to reduce the blank value stemmed from the serum component.

The light shielding subsance can be provided in the form of a independent coating layer between the sharing sheet and the reaction sheet by applying therebetween a dispersion of the light shielding substance in a binder such as a gelatin. In this case, the light shielding layer preferably has a thickness ranging from 100 µm to 300 µm.

The multilayer analytical element of the present invention can be enclosed with a frame and employed in the form of a slide for fluoroimmunoassay.

The frame has an opening for introduction (or application) of the sample liquid on one side, as well as an opening for the measurment of fluorescence strength on another side. In more detail, the frame comprises a member having the opening for the introduction and a member having the opening for the fluorometric measurement. Both members are united through an adhesive or direct fusion therewith.

The slide can be prepared as follows: A plurality of parts constituting the frame are independently produced from a injection-mouldable plastic material such as polyethylene, polyvinyl chloride, polyethylene terephthalate or polystyrene, and the so prepared parts are combined with an adhesive agent or an adhesive tape after receiving the analytical element therein. The adhesion of the frame parts can be done by fusing the respective surfaces under application of suppersonic waves to prepare a slide. Otherwise, whole or a part of the frame members are cut out of appropriate sheets or plates such as plastic plates, papers laminated with plastic sheets, or metal plates or sheets to correspond to the shape of the analytical element, and then these frame members are combined through adhesion to form a frame.

As mentioned hereinbefore, the frame comprises a couple of frames; one has an opening for introduction of the sample liquid, and another has an opening for measurement of the fluorescence strength. The opening for the liquid introduction (or application) or fluorometric measurement generally has a diameter in the range of from 3 mm to 1 cm.

Where the multilayer analytical element of the invention is enclosed with a frame and thus employed in the form of a slide, the space between a surface of the frame member facing a sharing sheet and the surface of the sharing sheet is preferably not more than 1 mm. This is because at least 70% of a spotted or applied sample liquid ought to be introduced and spreaded into the analytical element.

The analyte which can be assayed by the use of the multilayer analytical element of the invention is a high molecular or low molecular substance present in a biochemical component-containing liquid or a body fluid such as blood, plasma, serum, spinal fluid and saliva.

Representative examples of the analyte are as follows:

(1) Polypeptides, proteins, polysaccharides, nucleic acids and their complexes.

Complexes are bacilla, viruses, cell membranes, genes and nuclei. The molecular weight of these complexes is at least 5,000, generally 10,000 or more. Polypeptides and proteins generally have a molecular weight of from 5,000 to 5,000,000, ordinarily 20,000 to 1,000,000. In the case of peptide hormones, the molecular weight generally ranges from 5,000 to 60,000.

(a) Proteins

(i) Simple proteins: protamine, albumin, and globulins (e.g., α-globulin, β-globulin — particularly immunoglobulin—, IgG, IgA, IgE, IgM, IgD), screloproteins (structural proteins such as collagen, elastin, and actin).

(ii) Conjugated proteins: mucoprotein, chromoprotein, lipoprotein, nucleoprotein, glycoprotein, and phosphoprotein.

(iii) Other proteins including enzymes and complements

(b) Peptide hormones

insulin, glucagon, somatotropin, corticotropin, gonadotropin, gastrin, secretin, pituitary hormone, etc., precursors thereof and metabolites thereof

(c) Microorganism-originated antigenic polysaccharides

coccus (Streptococcus, Staphylococcus), bacillus (bacillus anthracis, bacillus subtilis, clostridium tetani), actinomyces (Actinomyces), eumycetes (nocardia, aspergillus, candida), richettsia (typhus, tutugamushi disease, Rocky Mountain spotted fever, Q-fever), viruses (herpes, adenoid vegitation, albo, hepatitis), spirochaeta (syphilis, leptospira, treponema) and other pathogenic bacteria.

(2) Antigen low molecular weight substances

Antigenic low molecular weight substances having a molecular weight of generally from 100 to 2,000, ordinarily 125 to 1,000. Examples include the following drugs, agricultural chemicals, small peptides, amino acids, low molecular weight hormones, and metabolites thereof:

(a) Drugs

alkaloids (morphine, codeine, kinine, digoxine), 5- or 6-membered lactams (barbiturates), amino-alkylbenzenes (amphetamine, epinephrine, catecholamine), benzo-heterocyclic compounds (oxazepam, chlorpromazine), purines, (theophylline, caffeine), vitamins (A, B complex, C, D, E), prostaglandins, antibiotics (penicillin, tetracycline, cephalosporin), aminoglycosides (gentamycine, kanamycine), other drugs (methadone, meprobamate, lidocaine, griseofulvin), and metabolites of these drugs

(b) Agricultural chemicals

halogenated biphenyls, phosphoric acid esters, thiophosphates and metabolites thereof.

(c) Small peptides, amino acids, low molecular weight hormones, triiodothyronine, thyroxine ($T_4$), encepharine, bradykinine, angiotensine I and II, and metabolites thereof.

The multilayer analytical element of the present invention is further described hereunder by referring to the attached drawings.

Figure 1 is a sectional view showing the essential layer structure of a multilayer analytical element of the invention. There are arranged a spreading sheet 101, a sharing sheet 111, and a reaction sheet 121, in this order from the top. The reaction sheet 121 contains a predetermined amount of an antibody corresponding to the analyte. The antibody is physically adsorbed by the reaction sheet 121. The spreading sheet 101 contains a predetermined amount of a fluorescent-labelled antigen in which the antigen is the same as the antigen of analyte. Figure 1a is a perspective view of the multilayer analytical element shown in Figure 1.

Figure 2 is a vertical sectional view of an embodiment of a slide for fluoroimmunoassay according to the present invention, in which the numeral 251 indicates a frame having an opening X for introduction of a sample liquid and the numeral 252 indicates a frame having an opening Y for fluorometric measurement. The multilayer analytical element given in Figure 1 is enclosed with the frames 251, 252. Figure 2a is a top plane view of the slide shown in Figure 2; Figure 2b is a sectional view of a upper part of the slide taken along line I—I in Figure 2a; Figure 2c is a bottom plane view of the slide shown in Figure 2; and Figure 2d is a sectional view of a lower part of the slide taken along line I—I in Figure 2c.

Figure 3 is a vertical sectional view of another embodiment of a slide for fluoroimmunoassay according to the present invention, in which the numeral 351 indicates a frame having an opening X for introduction of a sample liquid and the numeral 352 indicates a frame having an opening Y for fluorometric measurement, and the numerals 353 and 354 indicate adhesive sheets. A multilayer analytical element consisting of a spreading sheet 301, a sharing sheet 311 and a reaction sheet 321 is enclosed with these frames. In Figure 3, the numeral 302 indicates a distributor having openings 303 for supplying a sample liquid. A sectional view and plane view of the distributor 302 are given respectively in Figures 3c and 3d. Figures 3a and 3b are respective sectional views of two frames.

Figure 4 is a vertical sectional view of a further embodiment of a slide for fluoroimmunoassay according to the present invention, in which the numeral 451 indicates a frame having an opening X for introduction of a sample liquid, the numeral 452 indicates a frame having an opening Y for fluorometric measurement, and the numerals 453 and 454 indicate adhesive sheets. A multilayer analytical element consisting of a spreading sheet 401, a sharing sheet 411, a reaction sheet 421 and a transparent or clear support 431 having vents 455 is enclosed with these frames, as illustrated in Figure 4. A distributor 402 having openings for supplying a sample liquid is provided between the spreading sheet 401 and the sharing sheet 411. Figures 4a and 4b are repsectively a sectional view and a plane view of the transparent support 431.

Figure 5 is a vertical sectional view of a still further embodiment of a slide for fluoroimmunoassay according to the present invention, in which the numeral 551 indicates a frame having an opening X for introduction of a sample liquid, the numeral 552 indicates a frame having an opening Y for fluorometric measurement, and the numerals 553 and 554 indicate adhesive sheets. A multilayer analytical element consisting of a spreading sheet 501, a sharing sheet 511, a reaction sheet 521 and a transparent support 531 having vents 503 is enclosed with these frames. Figures 5a and 5b are respectively a sectional view and a plane view of a distributor 502 having openings 503 for supplying a sample liquid. The distributor 502 is provided between the spreading sheet 501 and the sharing sheet 511. Figures 5c and 5d are respectively a sectional view and a plane view of the transparent support 531 having vents 503.

Figure 6 is a vertical sectional view of a still further embodiment of a slide for fluoroimmunoassay according to the present invention, in which the numeral 651 indicates a frame having an opening X for introduction of a sample liquid, the numeral 652 indicates a transparent frame having vents 655, and the numerals 653 and 654 indicate adhesive sheets. A multilayer analytical element consisting of a spreadng sheet 601, a distributor 602 having openings for supplying a sample liquid, a sharing sheet 611, and a reaction sheet 621 is enclosed with these frames. Figures 6a and 6b are respectively a sectional view and a top plane view of the frame 651 having an opening X for introduction of a sample liquid. Figures 6c and 6d are respectively a sectional view and a bottom plane view of the lower transparent frame 652 having vents 655.

Figure 7 is a vertical sectional view of a still further embodiment of a slide for fluoroimmunoassay according to the present invention, in which the numeral 751 indicates a frame having an opening X for introduction of a sample liquid, the numeral 752 indicates a frame having an opening Y for fluorometric measurement, and the numerals 753 and 754 indicate adhesive sheets. A multilayer analytical element consisting of a spreading sheet 701, a distributor 702, a sharing sheet 711, a light shielding sheet 741, a reaction sheet 721 and a transparent support 731 having vents is enclosed with these frames.

A procedure for quantitative assay of an analyte using a slide illustrated in Figure 5 is given below, for illustrating an exemplary procedure using an analytical slide of the present invention.

The spreading sheet 501 is prepared by immersing a pulp paper in a buffer solution containing a FITC-labelled analyte substance and then freeze-drying the so immersed paper. The dry thickness of the spreading sheet is 200 µm.

The distributor 502 is a polyethylene terephthalate (PET) sheet (thickness: 105 µm) having nine openings 503 (diameter: 240 µm).

The sharing sheet 511 is prepared from a glass fiber suspension containing red colored agarose (light shielding agent) and has a dry thickness of 450 µm.

The reaction sheet 521 is prepared as follows: A glass fiber suspension containing agarose particles onto the surface of which is fixed on IgG fraction of anti-rabbit IgG goat sera is processed to prepare a paper having a dry thickness of 350 µm. A dilute solution of a rabbit antiserum (analyte) is dropped on the so prepared paper, and the antiserum-incorporated paper is freeze-dried to obtain the reaction sheet.

The support 531 is a cellulose triacetate sheet (thickness: 135 µm) having vents 555 (diameter: 240 µm).

The procedure is as follows: To the opening X of the slide illustrated in Figure 5 is applied 150 µl. of an analyte(antibody)-containing serum, and then the slide is subjected to incubation at 35°C for 15 min. The sample liquid is spread over the spreading sheet 501 immediately upon the application of the sample liquid. In this stage, approximately 20 µl. out of the 150 µl. of the sample liquid remains in the spreading sheet 501. The sample liquid gradually permeates the sharing sheet 511 and the reaction sheet 521 through the openings 503 of the distributor 502. Finally, the sample liquid is shared uniformly over the whole portion. With the progress of the sharing of the same liquid, the FITC-labelled substance contained in the spreading sheet 501 is dissolved in the serum solution, and then supplied into the sharing sheet 511 and the reaction sheet 521 together with a buffer agent and other substances. The so supplied substances are subjected to a competitive antigen-antibody reaction in the reaction sheet in the course of the incubation. In this stage, a free FITC-labelled substance is shared between the reaction sheet 521 and the sharing sheet 511. Thus, approximately 60% of the free FITC-labelled substance remains in the sharing sheet 511. The ratio of the free FITC-labelled substance shared between the sharing sheet 511 and the reaction sheet 521 finally corresponds to a ratio of the liquid holding capacity therebetween.

After the incubation is complete, exciting rays of wavelength 498 nm are impinged upon the reaction sheet 521 through the support 531 at an angle of incidence 45° via the opening Y. At the same time, a fluorescence emitted by the FITC-labelled substance (free FITC-substance shared as above + reacted FITC-labelled substance + antibody complex-originated substance) contained in the reaction sheet 521 is caught at an angle of 90° for the measurement. In this measurement, the FITC-labelled substance contained in the sharing sheet 511 does not emit a fluorescence because the red colored agarose contained in the sheet absorbs the exciting rays.

Independently, a calibration curve is prepared by a use of a calibration-purpose serum containing a predetermined amount of the analyte substance. By referring the fluorescence strength measured on the unknown amount of the analyte (antigen) to the calibration curve, the amount of the analyte can be determined.

As described above, the assay according to the present invention does not involve measurement of whole fluorescence strength of an antigen-antibody reaction mixture that is contained in the element without having been subjected to B/F separation and that thus includes all of bound fluorescent-labelled complex (B) produced on reaction as well as all of free fluorescent-labelled substance (F), the measurement of such whole fluorescence strength being described in Japanese Patent Provisional Publication No. 53(1978)—79024. Moreover, the assay according to the present invention does not involve measurement on only a free fluorescent-labelled substance (F) separated by B/F separation as in the prior application of the present applicant.

In contrast, the assay according to the present invention comprises measurement of fluorescence strength of a bound fluorescent-labelled complex (B) separated through the B/N separation based on the sharing. For this reason, possible noise is greatly reduced in the measurement according to the present invention, and therefore the assay according to the present invention is prominently advantageous in comparison with the assays of the above-cited two prior art disclosures, particularly the assay disclosed in Japanese Patent Provisional Publication No. 53(1978)—79024.

Moreover, the assay according to the present invention can be applied to any competitive antigen-antibody reaction so far as said reaction is detectable in fluoroimmunoassay, while the art disclosed in Japanese Patent Provisional Publication No. 53(1978)—79024 can be utilized only in a detection system having the aforementioned specific relationship on the fluorescence strength and accordingly is applicable only to antigen-antibody reaction involving thyroid hormones. In other words, according to the present invention, quantitative assay on analyte can be accomplished not only in a system in which a fluorescence strength of a bound fluorescent-labelled complex (B) is weaker than that of a free fluorescent-labelled substance (F), but also in a system in which there is no difference in the fluorescence strength between the bound fluorescent-labelled complex (B) and the free fluorescent-labelled substance (F). Whereas, the art disclosed in Japanese Patent Provisional Publication No. 53(1978)—79024 is not applicable to these two cases.

The present invention is further described by the following examples.

Example 1

(1) Preparation of a Fluorescein-labelled Thyroxine (FITC—$T_4$).

FITC—$T_4$ having the following structure was prepared by a series of the procedures i), ii) and iii) given below.

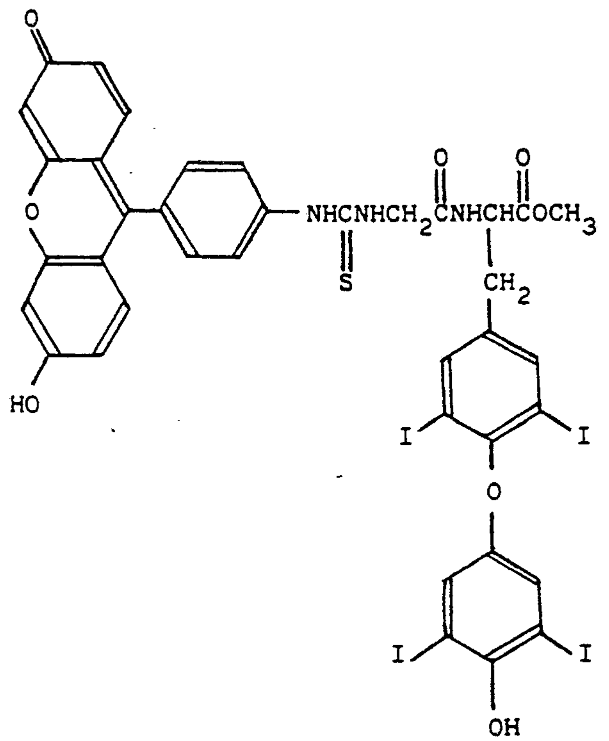

i) Preparation of t-butoxycarbonylglycyl thyrozine methyl ester (Boc-Gly-$T_4$-$OCH_3$).

In 5 ml. of tetrahydrofuran (THF), 329 mg. (1.21 mmol) of t-butoxycarbonylglycine succinyl ester (Boc-Gly-OSu) was dissolved. Independently, 1.0 g. (1.21 mmol) of thyroxine methyl ester hydrochloride is dissolved in 15 ml. of THF, and to this solution was added 170 µl. (1.21 mmol) of triethylamine ($Et_3N$) in 3 ml. of THF.

To the Boc-Gly-OSu solution was added under ice-cooling the thyroxine methyl ester solution. The resulting mixture was allowed to stand to reach room temperature, and then stirred for 1.5 h. The reaction liquid was allowed to stand overnight, and to this were added 25 ml. of distilled water and 50 ml. of ethyl acetate for extraction with the ethyl acetate. The extraction with 25 ml. of ethyl acetate was repeated three times. The ethyl acetate extracts were combined and dried over anhydrous magnesium sulfate. The dry extract was concentrated in a rotary evaporator and subjected to gel chromatography using a column filled with Sephadex LH-20® (tradename of Pharmacia Corp.). In this procedure, a mixture of acetone and methanol in a volume ratio of 4:1 was employed as the solvent. After confirming by TLC, the fractions containing the desired product were collected and concentrated under reduced pressure to obtain a crystalline product. There was thus obtained 950 mg. (yield 82.5%) of the desired product.

ii) Removal of BOC

950 mg. (1 mmol) of Boc-Gly-$T_4$-$OCH_3$ prepared in the above i) was dissolved in 10 ml. of trifluoroacetic acid. The resulting mixture was stirred for 10 min. under ice-cooling, and subjected to evaporation under reduced pressure at a temperature of below 40°C. The residue was subjected to gel chromatography under the same conditions as in the above i) to collect fractions containing the desired product. Upon crystallization, there was obtained 700 mg. (yield 82.5%) of the desired product.

iii) Preparation of Fluorescein isothiocyanic acid glycylthyroxine methyl ester (FITC-Gly-$T_4$-$OCH_3$)

320 mg. (0.33 mmol) of Gly-$T_4$-$OCH_3$ trifluoroacetic acid salt obtained in the above ii) and 47 µl. (0.33 mmol) of triethylamine were dissolved in 5 ml. of methanol. The resulting solution was added to 130 mg. (0.33 mmol) of FITC in 45 ml. of methanol. After reaction was carried out for 34 h. at room temperature, the solvent was removed under reduced pressure at a temperature below 40°C. The residue was subjected to gel chromatography under the same conditions as in the above i) to collect fractions containing the desired product. Upon crystallization, there was obtained 290 mg. (yield 70%) of an orange colored crystalline product.

The so obtained product was identified by Elementary analysis, NMR spectlrum and Mass spectrum.

FITC—$T_4$ prepared as above was a labelled substance which was able to participate in a competitive antigen-antibody reaction in conjunction with thyroxine ($T_4$). In this antigen-antibody reaction, it was noted that a bound FITC-labelled antigen-antibody complex (B) showed a fluorescence strength approximately twice as much as that shown by a free fluorescent-labelled substance.

(2) Preparation of Multilayer Analytical Element

i) Spreading sheet

A filter paper 5C (tradename, produced by Toyo Filter Paper Co., Ltd., Japan) was cut into a disk having a diameter of 15 mm.

Independently, FITC-$T_4$ prepared in the above i) was dissolved in 0.5 N aqueous sodium hydroxide solution in a concentration of 100 μg/ml. After 1 min., the resulting solution was diluted with 0.1 M glycine buffer solution (pH 9) containing 0.1% BSA to obtain a solution in a concentration of 1.28 μg/ml. The so diluted solution was mixed with 40 mM aqueous 8-anilino-1-naphtalene-sulfonic acid solution in a volume ratio of 1:1, and 20 μl. of the resulting mixture was dropped on the above-mentioned filter paper disk. Immediately after the mixture was permeated into the filter paper disk, the disk was freeze-dried in a conventional manner to obtain a spreading sheet having a dry thickness of 200 μm.

ii) Distributor

A transparent PET film (thickness: 105 μm) was cut into a disk having a diameter of 15 mm. The disk was then provided with 9 openings (diameter: 230 μm) for introduction of a sample liquid to obtain a distributor.

iii) Sharing sheet

A glass fiber filter paper (Glass Fiber Filter Paper GA-100, produced by Toyo Filter Paper Co., Ltd., Japan) was cut into a 10 mm × 5 mm piece, and then dispersed in water. After addition of red colored agarose, the glass fiber dispersion was processed to obtain a sharing sheet.

iv) Reaction sheet

Reaction sheets (I), (II), and (III) were prepared by the methods given below. Each of the reaction sheets (I) and (II) was a constituent sheet of a multilayer analytical element of the present invention, while the reaction sheet (III) was a constituent sheet of a comparison multilayer analytical element. Since the comparison multilayer analytical element did not comprise a sharing sheet, the reaction sheet (III) was prepared to have a thickness corresponding to a thickness of an assumed thickness of a sharing sheet in addition to that of the reaction sheet (I) or (II).

*Reaction sheet (I)*

A glass fiber filter paper (Glass Fiber Filter Paper GA-100, produced by Toyo Filter Paper Co., Ltd., Japan) was dispersed in water. Independently, an anti-$T_4$ rabbit serum was fixed to surfaces of agarose particles in a conventional manner and the so treated agarose particles were mixed with the above-mentioned aqueous dispersion. The resulting mixture was processed and dried to prepare a reaction sheet (I) having a dry thickness of 350 μm.

*Reaction sheet (II)*

An aqueous glass fiber dispersion was prepared in the same manner as above. Independently, an IgG fraction of an anti-rabbit IgG goat serum was fixed to surfaces of agarose particles in a conventional manner and the so treated agarose particles were mixed with the above-mentioned aqueous dispersion. The resulting mixture was processed and dried to prepare a sheet. A diluted antibody-$T_4$ rabbit serum was dropped on the sheet and permeated thereinto. The sheet was then freeze-dried to prepare a reaction sheet (II) having a dry thickness of 350 μm.

*Reaction sheet (III)*

A reaction sheet (III) was prepared in the same manner as in the preparation of the reaction sheet (I) so as to contain the antibody in the same amount based on the surface area, but to have a dry thickness of 800 μm.

v) On each of the above-mentioned reaction sheets (I) and (II) were laminated the aforementioned spreading sheet and sharing sheet in the structure as illustrated in Figure 5 to prepare a multilayer analytical element (I) and a multilayer analytical element (II).

For comparison, a multilayer analytical element (III) was prepared in the same manner, using the reaction sheet (III) but not comprising a sharing sheet.

(3) Measurement on Thyroxyine ($T_4$)

On a spreading sheet of each of the multilayer analytical elements were dropped thyrozine ($T_4$)-containing serums for calibration purpose (prepared by treating a serum with active carbon to obtain a $T_4$ free serum and adding a predetermined amount of thyroxine to the serum) of different predetermined concentrations. The multilayer analytical element was incubated at 35°C for 15 min. After the incubation was complete, reflective fluorometric measurement was done on the side of the reaction sheet. The reflective fluorometric measurement was carried out at exciting rays of a wavelength 495 nm and a fluoroscence of a wavelength 525 nm by the use of a fluorophotometer 650—10(S) (manufactured by Hitachi, Ltd., Japan).

The results are given in Table 1 in terms of reflective fluoroscence strength (mV).

TABLE 1

| | $T_4$ Concentration (μg/dl) | | | | | |
|---|---|---|---|---|---|---|
| Element | 0 | 2 | 4 | 8 | 12 | 20 |
| I | 4.33 | 4.21 | 4.04 | 3.50 | 2.92 | 1.67 |
| II | 4.65 | 4.49 | 4.30 | 3.71 | 3.06 | 1.70 |
| III | 4.93 | 4.85 | 4.75 | 4.40 | 4.03 | 3.23 |

As is apparent from the results in Table 1, each of the multilayer analytical elements (I) and (II) comprising a sharing sheet according to the present invention showed a change of the fluorescence strength in relation to a change of the $T_4$ concentration. In contrast, although the comparison multilayer analytical element (III) comprising no sharing sheet likewise showed a change of the fluorescence strength in relation to a change of the $T_4$ concentration, the change of the fluoroscence strength was at a reduced level. This reduced level in the fluorescence strength change means that the multilayer analytical element (III) is disadvantageous in showing a measurement range inferior to (i.e., narrower than) that of the element (I) or (II) of the present invention.

For determining accuracy of the measurement, the measurement procedure was repeated ten times under the same conditions, using $T_4$ at a concentration of 8 μg/dl and a scatter of the measured values was checked through determination of the coefficient of variation (CV). The CV values on the multilayer analytical elements (I) and (II) were 4.5% and 4.8%, respectively, while the CV value on the multilayer analytical element (III) was 9.3%. Thus, it was observed that the CV value of the multilayer analytical element (III) was extremely far from the CV values of the multilayer analytical elements (I) and (II).

In consequence, it was apparent that the provision of a sharing sheet to the multilayer analytical element remarkably improves the measurement range and accuracy.

Example 2

The assay of $T_4$ was carried out in the same manner as in Example 1, using each of the multilayer analytical elements (I) and (III) and the compound having the structure given below as a fluorescent-labelled $T_4$.

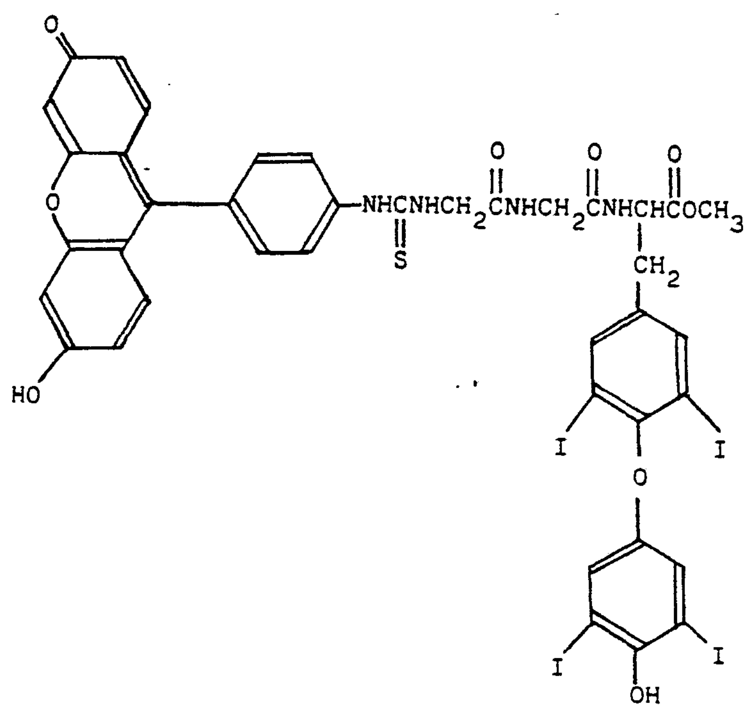

The above-illustrated compound was prepared in the same manner as in Example (1)—i), except that t-butoxycarbonylglycine succinyl ester was replaced with t-butoxycarbonylglycylglycine succinyl ester (Boc-Gly-Gly-OSu). This fluorescent-labelled $T_4$ did not show the increase of fluorescence strength upon subjected to an antigen-antibody reaction, which was different from the fluorescent-labelled substance

employed in Example 1. This means that the reaction using the above-mentioned labelled substance is not applicable to the assay described in Japanese Patent Provisional Publication No. 53(1978)—79024.

The results obtained on the multilayer analytical elements (I) and (III) are given in Table 2 in terms of reflective fluoroscence strength (mV).

TABLE 2

| | $T_4$ Concentration (μg/dl) | | | | | |
|---|---|---|---|---|---|---|
| Element | 0 | 2 | 4 | 8 | 12 | 20 |
| I | 4.83 | 4.70 | 4.59 | 4.16 | 3.81 | 2.86 |
| III | 5.81 | 5.85 | 5.77 | 5.83 | 5.79 | 5.84 |

As is apparent from the results in Table 2, the comparison multilayer analytical elements (III) did not show a change of the fluorescence strength in relation to a change of the $T_4$ concentration, and mere random fluorescence strength values were observed. This means that no calibration curve can be prepared in this assay system, and therefore a quantitative assay of $T_4$ is not accomplished. In contrast, the multilayer analytical element (I) comprising a sharing sheet showed a change of the fluorescence strength in relation to a change of the $T_4$ concentration, and this means that a calibration curve for the quantitative assay of $T_4$ can be successfully prepared.

In summary, the present invention utilizing a sharing phenomenon by the use of a sharing sheet enables to carry out a fluoroimmunoassay with little noise and high accuracy on a variety of competitive antigen-antibody reactions, irrespective of whether the bound fluorescent-labelled complex (B) prepared by a binding reaction with an antibody shows change or no change in the fluorescence strength, and further with no necessity of complicated B/F separation.

**Claims**

1. A multilayer analytical element for fluorometric assay of an analyte contained in an aqueous sample liquid, comprising;

a porous fibrous or non-fibrous spreading sheet containing a predetermined amount of a fluorescent-labelled analyte substance or analogue thereof;

a porous sharing sheet; and

a reaction sheet containing a predetermined amount of a protein fixed therein which is specifically bindable to the analyte; and the analogue thereof;

in which a ratio of the liquid-holding capacity between the porous sharing sheet and the reaction sheet is within the range of 10:1 to 0.5:1, and these three sheets are laminated in this order.

2. The multilayer analytical element as claimed in Claim 1, in which said analyte is an antigen, said protein is an antibody, and said fluorescent-labelled analyte substance or analogue thereof is a fluorescent-labelled antigen or analogue of the antigen.

3. The multilayer analytical element as claimed in Claim 1 or 2, in which said spreading sheet shows a higher spreading rate in the direction vertical to the sheet surface than the sharing sheet or reaction sheet does.

4. The multilayer analytical element as claimed in Claim 1 or 2, in which said sharing sheet is made of a porous fibrous material.

5. The multilayer analytical element as claimed in Claim 4, in which said sharing sheet contains fine granular particles independently distributed within the porous fibrous material.

6. The multilayer analytical element as claimed in Claim 4, in which said sharing sheet contains water-insoluble light-blocking material.

7. The multilayer analytical element as claimed in Claim 6, in which said light-blocking material is capable of absorbing either or both of a light for exciting the bound fluorescent-labelled complex (B) and a radiation emitted by the bound fluorescent-labelled complex (B).

8. The multilayer analytical element as claimed in Claim 6, in which said light-blocking material is material capable of scattering a light for exciting the bound fluorescent-labelled complex (B) or a radiation emitted by the bound fluorescent-labelled complex (B).

9. The multilayer analytical element as claimed in Claim 1 or 2, in which said reation sheet is made of a porous fibrous material.

10. The multilayer analytical element as claimed in Claim 9, in which said reaction sheet contains fine particles independently distributed within the porous fibrous material.

11. The multilayer analytical element as claimed in Claim 9, in which said porous fibrous material has a porosity of at least 60 percent in volume.

12. The multilayer analytical element as claimed in Claim 10, in which said fine particles are of water-insoluble polysaccharide or its derivative.

13. The multilayer analytical element as claimed in Claim 12, in which the surface of said fine particles are coated in part with a protein bindable to the analyte in the sample liquid through physical adsorption or chemical linkage.

14. The multilayer analytical element as claimed in Claim 1 or 2, in which a porous light-shielding sheet containing a material capable of absorbing or scattering either or both of a light for exciting the bound fluorescent-labelled complex (B) and a radiation emitted by the bound fluorescent-labelled complex (B) is provided between the sharing sheet and the reaction sheet.

15. The multilayer analytical element as claimed in Claim 1 or 2, in which a sheet having openings for introduction of the sample liquid in the range of 0.00001—0.05 $cm^2$ is provided between the spreading sheet and the sharing sheet for assisting substantially uniform sharing.

16. The multilayer analytical element as claimed in Claim 1 or 2, in which a light-transmissive, water-impermeable support is provided to the outer surface of the reaction sheet.

17. The multilayer analytical element as claimed in Claim 16, in which said support has at least one opening.

18. A slide for fluorometric assay of an analyte contained in an aqueous sample liquid, in which a multilayer analytical element as claimed in Claims 1 to 17 is provided in a frame having an opening for introduction of the sample liquid and an opening for enabling fluorometric measurement.

19. A slide for fluorometric assay as claimed in Claim 18, said frame comprises a member having an opening for introduction of the sample liquid and a member having an opening for enabling fluorometric measurement, being united through an adhesive material or direct fusion therebetween.

20. A slide for fluorometric assay as claimed in Claim 19, the frame member contains a coloring material with does not reflect a light for exciting the bound fluorescent-labelled complex (B).

21. A fluorometric assay of an analyte contained in an aqueous sample liquid which is characterized by employing a multilayer analytical element comprising the analytical features as claimed in Claims 1 to 17 and accomplishing a substantial separation of the bound fluorescent-labelled complex (B) from the free fluorescent-labelled analyte substance or analogue thereof (F) through sharing of the free fluorescent-labelled analyte substance or analogue thereof (F) between the reaction sheet and a space other than the reaction sheet.

**Patentansprüche**

1. Mehrschichtiges Analysen-Element zur fluorometrischen Analyse eines in einer wäßrigen Flüssigkeitsprobe enthaltenen Analyten, umfassend

ein poröses faserförmiges oder nicht-faserförmiges Ausbreitungsblatt, das eine vorher festgelegte Menge einer fluoreszenz-markierten Analyt-Substanz oder ein Analogon derselben enthält,

ein poröses Verteilungsblatt und

ein Reaktionsblatt, das eine vorher festgelegte Menge eines darin fixierten Proteins enthält, das an den Analyten und dessen Analogon spezifisch bindungsfähig ist, worin das Verhältnis des Flüssigkeits-Haltevermögens zwischen dem porösen Verteilungsblatt und dem Reaktionsblatt innerhalb des Bereichs von 10:1 bis 0,5:1 lieght und diese drei Blätter in der angegebenen Reihenfolge laminiert sind.

2. Mehrschichtiges Analysen-Element nach Anspruch 1, dadurch gekennzeichnet, daß der Analyt ein Antigen ist, das Protein ein Antikörper ist und die fluoreszenz-markierte Analyt-Substanz oder deren Analogen ein fluoreszenz-markiertes Antigen oder Analogon des Antigens ist.

3. Mehrschichtiges Analysen-Element nach Ansprüchen 1 oder 2, dadurch gekennzeichnet, daß das Ausbreitungsblatt eine höhere Ausbreitungsrate in der Richtung senkrecht zu der Blattoberfläche zeigt als das Verteilungsblatt oder das Reaktionsblatt.

4. Mehrschichtiges Analysen-Element nach Ansprüchen 1 oder 2, dadurch gekennzeichnet, daß das Verteilungsblatt aus einem porösen, faserförmigen Material hergestellt ist.

5. Mehrschichtiges Analysen-Element nach Anspruch 4, dadurch gekennzeichnet, daß das Verteilungsblatt feine granulare Teilchen enthält, die unabhängig innerhalb des porösen faserförmigen Materials verteilt sind.

6. Mehrschichtiges Analysen-Element nach Anspruch 4, dadurch gekennzeichnet, daß das Verteilungsblatt ein wasserunlösliches, lichtblockierendes Material enthält.

7. Mehrschichtiges Analysen-Element nach Anspruch 6, dadurch gekennzeichnet, daß das lichtblockierende Material befähigt ist, das Licht zur Erregung des gebundenen fluoreszenz-markierten Komplexes (B) oder eine von dem gebundenen fluoreszenz-markierten Komplex (B) emittierte Strahlung oder beide Strahlungsarten zu absorbieren.

8. Mehrschichtiges Analysen-Element nach Anspruch 6, dadurch gekennzeichnet, daß das lichtblockierende Material befähigt ist, das Licht zur Erregung des gebundenen fluoreszenz-markierten Komplexes (B) oder ein von dem gebundenen fluoreszenz-markierten Komplex (B) emittierte Strahlung zu streuen.

9. Mehrschichtiges Analysen-Element nach Ansprüchen 1 oder 2, dadurch gekennzeichnet, daß das Reaktionsblatt aus einem porösen, faserförmigen Material hergestellt ist.

10. Mehrschichtiges Analysen-Element nach Anspruch 9, dadurch gekennzeichnet, daß das Verteilungsblatt feine Teilchen enthält, die unabhängig innerhalb des porösen faserförmigen Materials verteilt sind.

11. Mehrschichtiges Analysen-Element nach Anspruch 9, dadurch gekennzeichnet, daß das poröse faserförmige Material eine Porosität von wenigstens 60 Vol.-% hat.

12. Mehrschichtiges Analysen-Element nach Anspruch 10, dadurch gekennzeichnet, daß die feinen Teilchen solche eines wasserunlöslichen Polysaccharids oder eines Derivats desselben sind.

13. Mehrschichtiges Analysen-Element nach Anspruch 12, dadurch gekennzeichnet, daß die Oberfläche der feinen Teilchen teilweise mit einem Protein beschichtet ist, das befähigt ist, mit dem Analyten in der Flüssigkeitsprobe vermittels physikalischer Adsorption oder chemischer Verknüpfung eine Bindung einzugehen.

14. Mehrschichtiges Analysen-Element nach Ansprüchen 1 oder 2, dadurch gekennzeichnet, daß ein poröses, lichtabschirmendes Blatt, das ein Material enthält, das zur Absorption oder Streuung von Licht zur Erregung des gebundenen fluoreszenz-markierten Komplexes (B) oder einer von dem gebundenen fluoreszenz-markierten Komplex (B) emittierten Strahlung oder beider Strahlungsarten befähigt ist, zwischen dem Verteilungsblatt und dem Reaktionsblatt angeordnet ist.

15. Mehrschichtiges Analysen-Element nach Ansprüchen 1 oder 2, dadurch gekennzeichnet, daß ein Blatt mit Öffnungen zum Einführen der Flüssigkeitsprobe im Bereich von 0,00001 bis 0,05 $cm^2$ zwischen dem Ausbreitungsblatt und dem Verteilungsblatt zur Unterstützung einer im wesentlichen gleichmäßigen Verteilung angeordnet ist.

16. Mehrschichtiges Analysen-Element nach Ansprüchen 1 oder 2, dadurch gekennzeichnet, daß ein lichtdurchlässiger, wasserundurchlässiger Träger auf der äußeren Oberfläche des Reaktionsblattes angebracht ist.

17. Mehrschichtiges Analysen-Element nach Anspruch 16, dadurch gekennzeichnet, daß der Träger wenigstens eine Öffnung aufweist.

18. Diapositiv zur fluorometrischen Analyse eines in einer wäßrigen Flüssigkeitsprobe enthaltenen Analyten, in dem ein mehrschichtiges Analysen-Element nach Ansprüchen 1 bis 17 in einem Rahmen mit einer Öffnung zur Einführung der Flüssigkeitsprobe und einer Öffnung für die Ermöglichung der fluorometrischen Messung angeordent ist.

19. Diapositiv zur fluorometrischen Analyse nach Anspruch 18, dadurch gekennzeichnet, daß der Rahmen ein Bauelement mit einer Öffnung zur Einführung der Flüssigkeitsprobe und ein Bauelement mit einer Öffnung für die Ermöglichung der fluorometrischen Messung umfaßt, die miteinander vermittels eines Klebstoffs oder durch direkte Verschmelzung zwischen beiden vereinigt sind.

20. Diapositiv zur fluorometrischen Analyse nach Anspruch 19, dadurch gekennzeichnet, daß das Rahmen-Bauelement ein färbendes Material enthält, das kein Licht zur Erregung des gebundenen fluoreszenz-markierten Komplexes reflektiert.

21. Fluorometrische Analyse eines in einer wäßrigen Flüssigkeitsprobe enthaltenen Analyten, dadurch gekennzeichnet, daß ein die analytischen Merkmale nach Ansprüchen 1 bis 17 aufweisendes Analysen-Element eingesetzt wird und eine wesentliche Trennung des fluoreszenz-markierten Komplexes (B) von der freien fluoreszenz-markierten Analyt-Substanz oder dem Analogon derselben (F) durch Verteilen der freien fluoreszenz-markierten Analyt-Substanz oder des Analogons derselben (F) zwischen dem Reaktionsblatt und einem anderen Raum als dem Reaktionsblatt erzielt wird.

**Revendications**

1. Un élément analytique multicouche pour un essai fluorométrique d'un analyte contenu dans un échantillon aqueux de liquide et comprenant

une feuille poreuse fibreuse ou non-fibreuse de répartition qui contient une quantité prédéterminée d'une substance analyte marquée par fluorescence ou an analogon de celle-ci,

une feuille poreuse qui en fait partie, et

une feuille de réaction qui contient une quantité prédéterminée d'une protéine y fixée et adaptée à être liée spécifiquement à l'analyte ou à son analogon, le rapport de la capacité retentive pour liquide entre la feuille poreuse qui en fait partie et la feuille de réaction se situant entre 10:1 et 0.5:1 et les trois feuilles étant laminées dans l'ordre indiqué.

2. L'élément analytique multicouche selon la revendication 1, dans lequel l'analyte est un antigène, la protéine étant un anti-corps et la substance analyte marquée par fluorescence ou son analogon étant un antigène marqué par fluorescence ou un analogon de l'antigène.

3. L'élément analytique multicouche selon les revendications 1 ou 2, dans lequel la feuille de répartition montre une vitesse de répartition plus grande en direction verticale par rapport à la feuille surface que la feuille faisant partie et la feuille de réaction.

4. L'élément analytique multicouche selon les revendications 1 ou 2, dans lequel la feuille qui en fait partie est fait d'un matériel poreux fibreux.

5. L'élément analytique multicouche selon la revendication 4; dans lequel la feuille qui en fait partie contient des particules fines granulées qui sont distribuées d'une manière indépendante dans le matériel poreux fibreux.

6. L'élément analytique multicouche selon la revendication 4, dans lequel la feuille qui en fait partie contient un matériel insoluble dans l'eau et bloquant la lumière.

7. L'élément analytique multicouche selon la revendication 6, dans lequel le matériel bloquant la lumière est capable à absorber ou la lumière pour exciter le complexe attaché marqué par fluorescence (B) ou une radiation émise par la complexe attaché marqué par fluorescence (B) ou les deux types de radiation.

8. L'élément analytique multicouche selon la revendication 6, dans lequel le matériel bloquant la lumière est un matériel capable à disperser la luminière pour exciter le complexe attaché marqué par fluorescence (B) ou la radiation émise par le complexe attaché marqué par fluorescence (B).

9. L'élément analytique multicouche selon la revendication 1 ou 2, dans lequel la feuille de réaction est faite d'un matériel poreux fibreux.

10. L'élément analytique multicouche selon la revendication 9, dans lequel la feuille de réaction contient des particules fines distribuées d'une manière indépendate dans le matériel poreux fibreux.

11. L'élément analytique multicouche selon la revendication 9, dans lequel le matériel poreux fibreux a une porosité de 60 pourcent au moins en volume.

12. L'élément analytique multicouche selon la revendication 10, dans lequel les particules fines sont d'un polysaccharide insoluble dans l'eau ou de son dérivé.

13. L'élément analytique multicouche selon la revendication 12, dans lequel la surface des particules fines est courverte en partie d'une proteine adaptée à être attachée à l'analyte dans l'échantillon de liquide par adsorption physique ou par combination chimique.

14. L'élément analytique multicouche selon la revendications 1 ou 2, dans lequel est prévu entre la feuille qui en fait partie et la feuille de réaction, une feuille poreuse à effet d'écran contre la lumière qui contient un matériel adapté à absorber ou à disperser une lumière pour exciter le complexe attaché marqué par fluorescence (B) ou la radiation émise par le complexe attaché marqué par fluorescence (B) ou les deux types de radiation.

15. L'élément analytique multicouche selon les revendications 1 ou 2 dans lequel est prévu entre la feuille de répartition et la feuille qui en fait partie une feuille ayant des ouvertures pour l'introduction d'un échantillon de liquide dans l'intervalle de 0.00001—0.05 $cm^2$ pour assister essentiellement la participation uniforme.

16. L'élément analytique multicouche selon les revendications 1 ou 2, dans lequel un support translucide étanche à l'eau est prévu à la surface extérieure de la feuille de réaction.

17. L'élément analytique multicouche selon la revendication 16, dans lequel le support est muni d'une ouverture au moins.

18. Un diapositive pour un essai fluorométrique d'un analyte contenu dans un échantillon aqueux de liquide dans lequel est prévu un élément analytique multicouche selon les revendications 1 à 17 dans un cadre ayant une ouverture pour y introduire l'échantillon de liquide et une ouverture pour permettre le mesurage fluorométrique.

19. Un diapositive pour un essai fluorométrique selon la revendication 18, dans lequel le cadre comprend un élément ayant une ouverture pour y introduire l'échantillon de liquide et un élément ayant une ouverture pour permettre le mesurage fluorométrique, ces éléments étant liés par une matière adhésive our par une fusion directe.

20. Un diapositive pour un essai fluorométrique selon la revendication 19, dans lequel l'élément de cadre contient une matière colorante qui ne reflète pas une lumière pour exciter le complexe attaché marqué par fluorescence (B).

21. Un essai fluorométrique d'un analyte contenu dans un échantillon aqueux de liquide caractérisé par les mesures suivants: employer un élement analytique multicouche comprenant les caractéristiques analytiques selon les revendications 1 à 17, et effectuer une séparation essentielle entre le complexe marqué par fluorescence attaché (B) et la substance analyte marquée par fluorescence or son analogon (F) en distribuant la substance analyte libre marquée par fluorescence or son analogon (F) entre la feuille de réaction et un espace autre que la feuille de réaction.

FIG.1

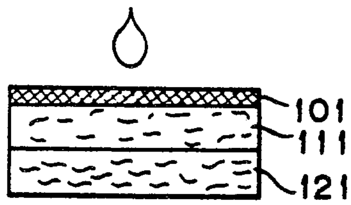


FIG.1a

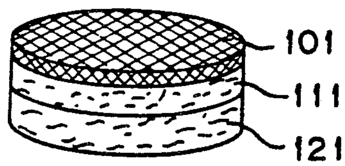


FIG.2

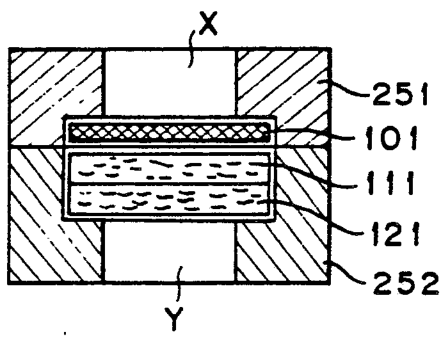


FIG.2a

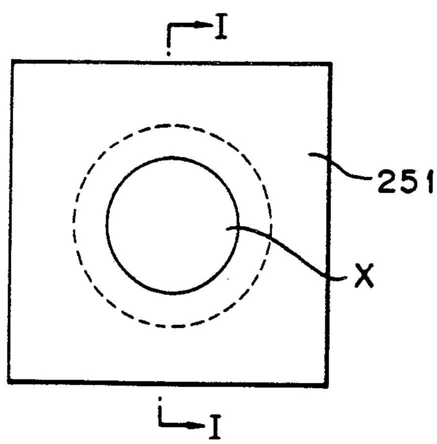


FIG.2b

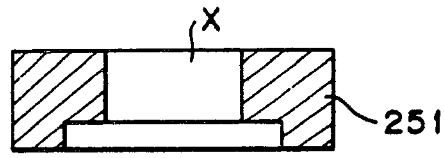


FIG.2c

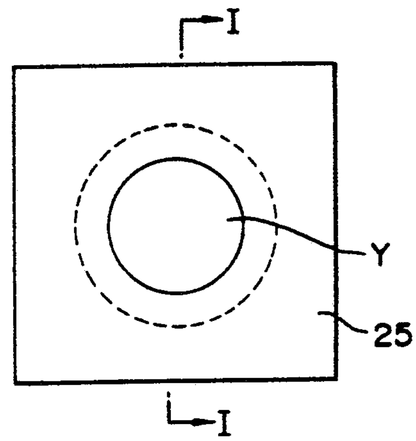


FIG.2d

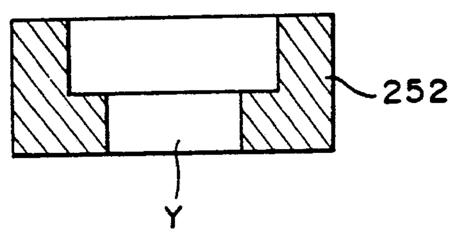

FIG.3

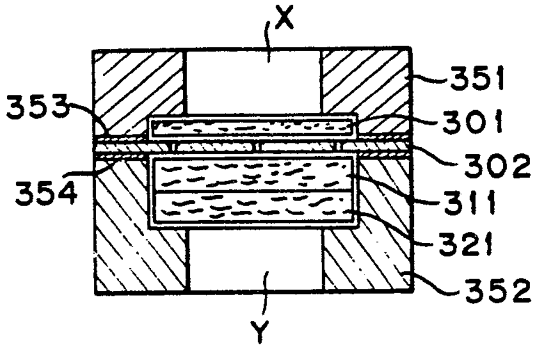


FIG.3a

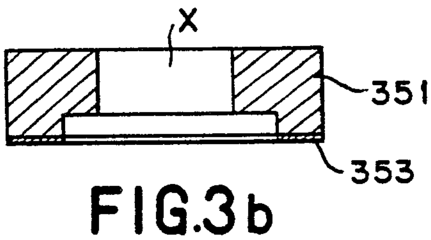


FIG.3b

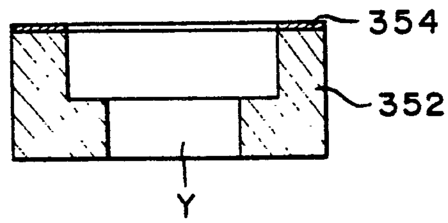


FIG.3c

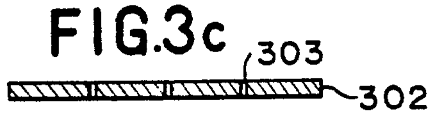


FIG.3d

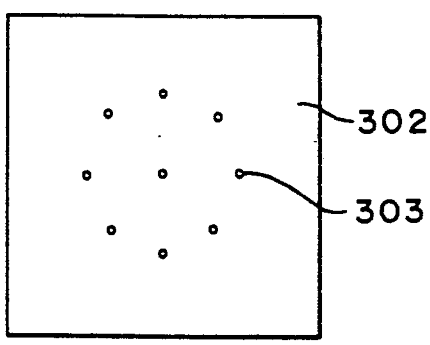


FIG.4

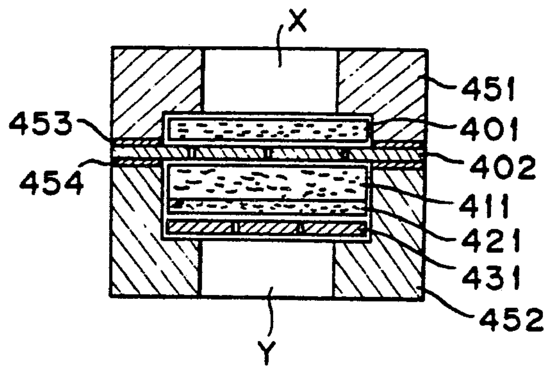


FIG.4a

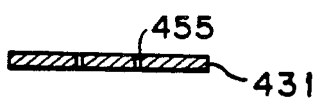


FIG.4b

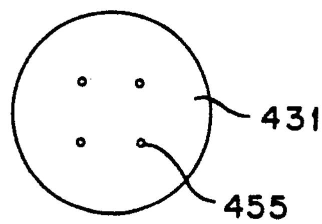


FIG.5

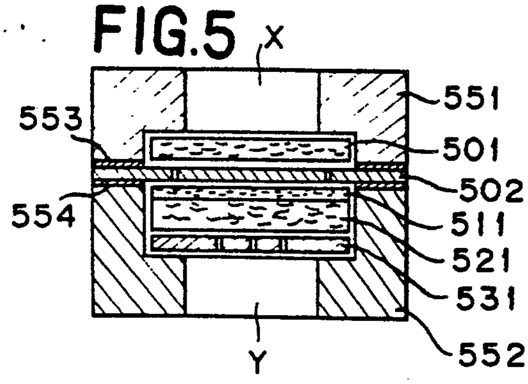

FIG.5a

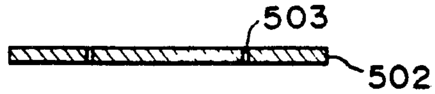


FIG.5b

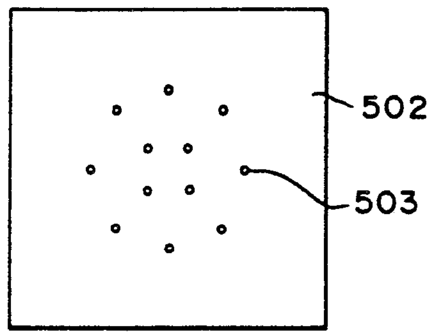


FIG.5c

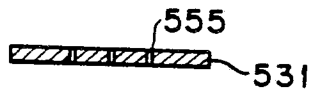


FIG.5d

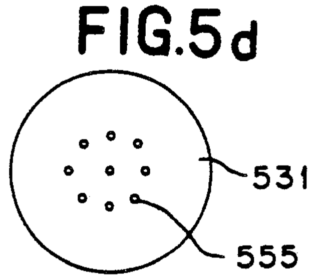


FIG.6

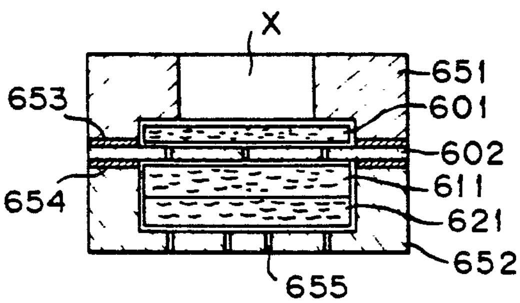


FIG.6a

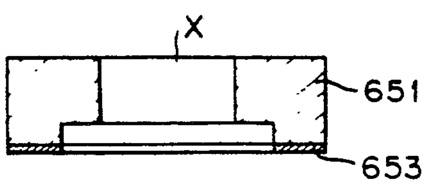


FIG.6b

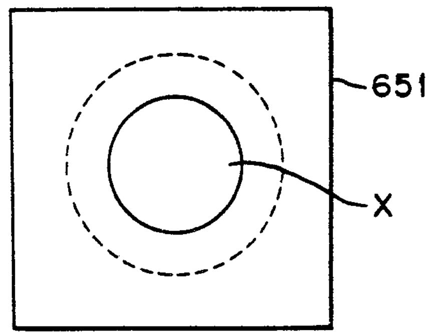


FIG.6c

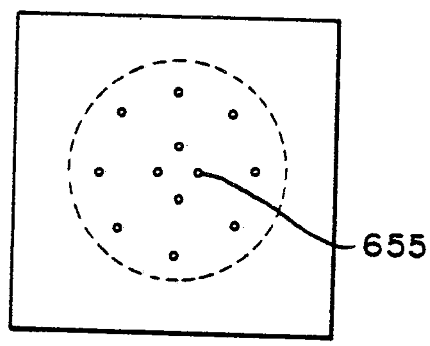


FIG.6d

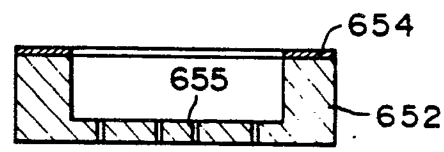


FIG.7